# EUROPÄISCHE PATENTANMELDUNG

(11) **EP 2 087 840 A1**
(43) Veröffentlichungstag der Anmeldung: **12.08.2009**
(21) Anmeldenummer: 08151294.9
(22) Anmeldetag: 11.02.2008
(51) Int. Cl.: A61B 5/15

(54) **Vorrichtung und Verfahren zur Körperflüssigkeitsentnahme**

(71) Anmelder: F.HOFFMANN-LA ROCHE AG, 4070 Basel (CH); Roche Diagnostics GmbH, 68305 Mannheim (DE)
(72) Erfinder: List, Hans, 64754 Hesseneck-Kailbach (DE); Rödel, Wolfgang, 69123 Heidelberg (DE)
(74) Vertreter: Pfiz, Thomas

(57) **Zusammenfassung**

Die Erfindung betrifft eine Vorrichtung zur Körperflüssigkeitsentnahme mit einem in die Haut eines Körperteils (17) einstechbaren Stechelement (14), das eine Aufnahmestruktur (18) für beim Hauteinstich gewonnene Körperflüssigkeit aufweist, und einem Stechantrieb (12) für eine Vorwärts- und Rückziehbewegung des Stechelements (14), wobei die Dauer der Rückziehbewegung länger als die Dauer der Vorwärtsbewegung ist. Erfindungsgemäß ist der Stechantrieb (12) dazu ausgebildet, das Stechelement (14) in einer ersten Rückziehphase (R1) der Rückziehbewegung mit einer maximalen Rückziehgeschwindigkeit von mehr als 0,02 m/s zu bewegen.

## Beschreibung

Die Erfindung betrifft eine Vorrichtung zur Körperflüssigkeitsentnahme mit einem in die Haut eines Körperteils einstechbaren Stechelement, das eine (insbesondere als Kapillare wirksame) Aufnahmestruktur für beim Hauteinstich gewonnene Körperflüssigkeit aufweist, und einem Stechantrieb für eine Vorwärts- und Rückziehbewegung des Stechelements, wobei die Dauer der Rückziehbewegung (vorzugsweise um ein Vielfaches) länger als die Dauer der Vorwärtsbewegung ist. Die Erfindung betrifft weiter ein entsprechendes Verfahren zur Entnahme von Körperflüssigkeit.

Für Blutzuckertests wurde bereits vorgeschlagen, eine automatische Probenentnahme aus der Haut durch einen Einstich eines Stechelements dadurch zu bewerkstelligen, das die Rückziehbewegung deutlich langsamer erfolgt als die Vorwärtsbewegung, so dass eine für den Nachweis ausreichende Probenmenge zuverlässig gewonnen werden kann. Die Position des Übergangs von schneller zu langsamer Bewegung sollte dabei nur so tief im Gewebe liegen, das eine in das Stechelement eingearbeitete Aufnahmestruktur noch sicheren Kontakt zur austretenden Flüssigkeit bekommt. In der WO 2007/073870 ist ein Stechsystem beschrieben, mit welchem die Übergangsposition trotz variabler Stechtiefe mit allerdings erheblichem technischem Aufwand konstant gehalten werden kann.

Ausgehend hiervon liegt der Erfindung die Aufgabe zugrunde, die im Stand der Technik vorgeschlagenen Systeme und Verfahren weiter zu verbessern und mit begrenztem baulichen Aufwand eine zuverlässige Probengewinnung zu gewährleisten und dabei auch die Schmerzhaftigkeit der Prozedur zu reduzieren.

Zur Lösung dieser Aufgabe wird die in den unabhängigen Patentansprüchen angegebene Merkmalskombination vorgeschlagen. Vorteilhafte Ausgestaltungen und Weiterbildungen der Erfindung ergeben sich aus den abhängigen Ansprüchen.

Die Erfindung geht von dem Gedanken aus, den Sammelbeginn auf eine Zwischenposition unter der Haut zu legen, die mit sehr hoher Geschwindigkeit angefahren werden kann, und die um eine feste Rückziehstrecke hinter der gewählten tiefsten Einstichposition liegt. Dementsprechend wird erfindungsgemäß vorgeschlagen, dass der Stechantrieb dazu ausgebildet ist, das Stechelement in einer ersten Rückziehphase der Rückziehbewegung mit einer maximalen Rückziehgeschwindigkeit von mehr als 0,02 m/s zu bewegen. Durch diese Maßnahme kann die maximale Stechtiefe entsprechend den individuellen Hauteigenschaften so gewählt werden, dass genügend Blutkapillaren durch den Stich geöffnet werden, während diese besonders schmerzintensive Phase durch die schnelle erste Rückbewegung auf ein Minimum reduziert wird. Überraschend hat sich gezeigt, dass oberhalb einer Geschwindigkeitsschwelle von 0,02 m/s die Schmerzreduzierung durch den raschen Nadelrückzug in weniger empfindliches Gewebe besonders effektiv wird, und dass ein solches Stechprofil den Sammelerfolg besser gewährleistet. In diesem Zusammenhang ist hervorzuheben, dass die Rückbewegung insgesamt deutlich länger dauern sollte als die Vorwärtsbewegung, so dass für die unmittelbare Körperflüssigkeitsentnahme während des Stechvorgangs im Vergleich zu einer einfachen Stechhilfe ein deutlich komplexeres Stechprofil realisierbar ist.

Gemäß einer bevorzugten Ausgestaltung wird das Stechelement während der ersten Rückziehphase um eine definierte erste Teilstrecke vorzugsweise von ca. 0,5 mm aus der tiefsten Einstechposition in eine unter der Hautoberfläche liegende Zwischenposition zurückgezogen wird. Die Hautoberfläche kann dabei durch eine entsprechende Referenzposition beispielsweise über eine Positionierung für das Körperteil oder einen Hautdetektor oder eine vorgegebene Stechtiefe vorrichtungsseitig bestimmt sein. Weiterhin kann durch den konstanten ersten Rückzugweg auf eine technisch aufwendige Bewegungssteuerung verzichtet werden, wobei ein harmonischer Bewegungsablauf im Bereich der Richtungsumkehr der Nadel ohne schwingungskritische Anschlagstrukturen möglich wird.

Vorteilhafterweise ist der Bewegungsablauf so implementiert, dass das Stechelement während einer zweiten Rückziehphase um eine zweite Teilstrecke aus der Haut zurückgezogen wird, und dass die mittlere Geschwindigkeit des Stechelements in der zweiten Rückziehphase vorzugsweise vielfach kleiner ist als in der ersten Rückziehphase. Somit findet ein Geschwindigkeitswechsel im Bereich der Zwischenposition in der Haut statt, um einen hinreichenden Flüssigkeitszufluss in der Stichwunde zu gewährleisten.

Besonders günstig ist es, wenn die Dauer der Vorwärtsbewegung zwischen 0,3 bis 0,7 ms beträgt, während die Dauer der ersten Rückziehphase zwischen 0,3 und 1 ms liegen sollte.

Um den selbständigen Flüssigkeitseintritt in die Aufnahmestruktur in hinreichender Menge sicherzustellen, ist es vorteilhaft, wenn die Dauer der zweiten Rückziehphase nicht kürzer als 0,4 s ist und vorzugsweise 0,5 bis 0,8 s beträgt.

Besonders im Hinblick auf die Anwenderfreundlichkeit, die aufgrund der vor allem bei Blutzuckertests unabdingbaren Testhäufigkeit ein entscheidendes Kriterium darstellt, ist es von großem Vorteil, wenn der Bewegungsablauf des Stechelements über den Stechantrieb so gesteuert ist, dass die Verweildauer des Stechelements im eingestochenen Zustand in der Haut im Bereich zwischen 0,5 bis 1,5 s liegt. Der eingestochene Zustand kann dabei wiederum durch eine Referenzposition für die Hautoberfläche oder einen Hautdetektor vorrichtungsseitig vorgegeben sein.

Für ein benutzerbezogenes Sammelprofil sollte die maximale Einstichtiefe zwischen 1 und 2,5 mm einstellbar sein.

Vorteilhafterweise ist der Geschwindigkeitsverlauf bei der Rückbewegung des Stechelements nach Maßgabe einer veränderlichen Stechtiefe so angepasst, dass das Stechelement für eine vorbestimmte Verweilzeit vorzugsweise zwischen 0,5 und 1,5 s in der Haut eingestochen bleibt. Auf diese Weise ist eine individuelle Stechtiefenanpassung möglich, ohne dass die Zyklusdauern sich ständig ändern.

Für eine einfachere Bewegungssteuerung kann es vorteilhaft sein, wenn der Geschwindigkeitsverlauf bei der Rückbewegung des Stechelements unabhängig von der Stechtiefe vorgegeben ist.

Eine weitere Verbesserung hinsichtlich der Probengewinnung wird dadurch erzielt, dass die Geschwindigkeit des Stechelements in einer an die erste Rückziehphase anschließenden zweiten Rückziehphase im Wesentlichen konstant ist. Hierbei sollte die maximale Geschwindigkeit des Stechelements in der zweiten Rückziehphase zwischen 0,001 und 0,005 m/s betragen.

Für eine vereinfachte Handhabung in einem integrierten System ist ein mit Körperflüssigkeit aus der Aufnahmestruktur beaufschlagbares Testelement vorgesehen.

Um eine nachteilige Probenveränderung weitgehend auszuschließen, sollte die Gesamtdauer vom Beginn der Rückziehbewegung des Stechelements bis zur Beaufschlagung des Testelements mit der Körperflüssigkeit weniger als 5 s, vorzugsweise weniger als 1 bis 2 s betragen.

Ein weiterer ergänzender oder alternativer Erfindungsaspekt liegt darin, dass ein für den Nachweis eines Analyten in der Körperflüssigkeit ausgebildetes Testelement so angeordnet ist, dass die Transferzeit zur Übertragung der Körperflüssigkeit aus der Aufnahmestruktur auf das Testelement (20) weniger als 1,5 s, vorzugsweise weniger als 1 s und bevorzugt weniger als 0,5 s beträgt. Überraschend hat sich herausgestellt, dass die Einhaltung dieses Zeitfensters besonders wichtig für die Testqualität ist. Das Testelement kann direkt auf dem Stechelement angeordnet sein und gegebenenfalls über eine Fließstrecke fluidisch mit der Aufnahmestruktur verbunden sein. Möglich ist es auch, das Testelement separat, insbesondere körperlich getrennt von der Aufnahmestruktur anzuordnen und die Flüssigkeit durch eine geeignete Aktuation beispielsweise unter Strukturverformung zu übertragen. Beispielhaft wird hier auf die WO 2005/084530 und WO 2007/025713 Bezug genommen.

Vorteilhafterweise besitzt das Stechelement ein beim Hauteinstich schmerzarm in die Haut eindringendes scharfes Stechorgan, insbesondere eine einzelne Nadelspitze.

Um mögliche Hauteindellungen beim Einstich zu berücksichtigen, kann die Position der Hautoberfläche durch einen Hautdetektor erfasst und/oder mittels einer Positioniereinheit für das Körperteil festgelegt werden.

Die Probengewinnung wird dadurch wesentlich vereinfacht, dass in das Stechelement eine zumindest an einem distalen Endabschnitt beim Hauteinstich in Kontakt mit der Körperflüssigkeit bringbare, insbesondere rinnen- oder schlitzförmige Kanalstruktur eingebracht ist.

Ein weiterer vorteilhafter Erfindungsaspekt liegt darin, dass der Antriebsmechanismus die Vorwärtsbewegung und Rückziehbewegung während einer ersten Rückziehphase des Stechelements steuert, während der Antriebsmotor das Stechelement in einer zweiten Rückziehphase der Rückziehbewegung aus der Haut zurückzieht. Auf diese Weise kann ein günstiges Bewegungsprofil mit einfachen technischen Mitteln realisiert werden. Dies ist besonders wichtig für in großer Stückzahl benötigte Testgeräte. Dabei kann der Antriebsmechanismus effektiv auf die schnelle Bewegung konzipiert werden, und der für die langsame Restbewegung vorgesehene Motor kann kompakt und energiesparend ausgelegt werden.

Vorteilhafterweise versorgt der Antriebsmotor den Antriebsmechanismus zur selbsttätigen Bewegungssteuerung mit mechanischer Energie.

Eine weitere Verbesserung wird dadurch erzielt, dass der Antriebsmotor den Antriebsmechanismus zusammen mit dem Stechelement als Verbund in der zweiten Rückziehphase zurückzieht.

Eine baulich besonders vorteilhafte Ausgestaqltung sieht vor, dass der Antriebsmechanismus eine mittels einer Feder angetriebene Kurvensteuerung aufweist.

Gegenstand der Erfindung ist auch ein Verfahren zur Körperflüssigkeitsentnahme bei welchem durch einen Stechantrieb eine Vorwärts- und Rückziehbewegung eines Stechelements so gesteuert wird, dass das Stechelement in einer ersten Rückziehphase der Rückziehbewegung mit einer maximalen Rückziehgeschwindigkeit von mehr als 0,02 m/s zurück bewegt wird.

Im Folgenden wird die Erfindung anhand eines in der Zeichnung schematisch dargestellten Ausführungsbeispiels näher erläutert. Es zeigen:
- Fig. 1: eine Vorrichtung für Blutzuckertests mit einem mehrstufigen Stechantrieb in vereinfachter Schnittdarstellung;
- Fig. 2: ein Stechprofil beim Einsatz der Vorrichtung nach Fig. 1.

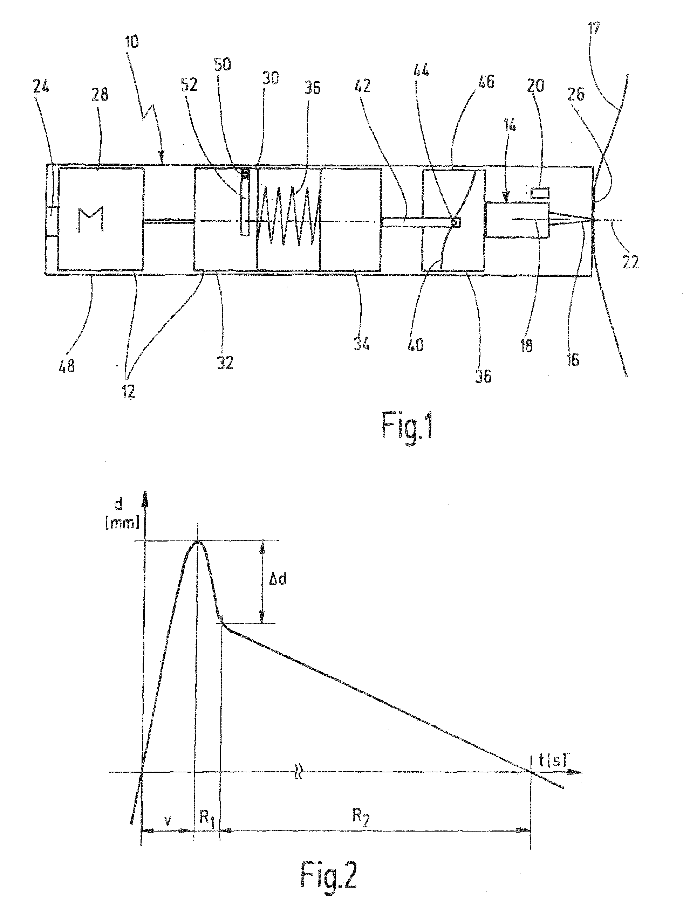

Die in Fig. 1 dargestellte Vorrichtung dient der Selbstentnahme einer Blutprobe durch einen Benutzer für Analysezwecke, insbesondere zur Blutzuckerkontrolle. Die Vorrichtung umfasst ein Handgerät 10 mit Stechantrieb 12 zur automatischen Handhabung eines als Einmalartikel zur Blutentnahme eingesetzten Stechelements 14.

Das Stechelement 14 ist als so genannter "Microsampler" zur Gewinnung einer kleinen Blutmenge aus einem Körperteil 17, insbesondere einer Fingerkuppe vorgesehen. Es kann als monolithisches einstückiges Formteil aus dünnem Edelstahlblech bestehen und eine distal angeformte Spitze 16 als Stechorgan zur Erzeugung einer Einstichwunde aufweisen. Ein mit seinem distalen Endabschnitt in den Bereich der Spitze 16 sich erstreckender rillen- oder schlitzförmiger Kapillarkanal 18 ermöglich die Aufnahme von Körperflüssigkeit (Blut und/oder Gewebeflüssigkeit) aus der Einstichwunde. Zum Nachweis der in der Körperflüssigkeit enthaltenen Zielsubstanz (Glucose) kann ein mit einer Testchemie versehenes Testelement 20 eingesetzt werden, welches nach dem Hauteinstich durch Herstellung einer geeigneten Fließverbindung mit Körperflüssigkeit aus der Aufnahmestruktur 18 beaufschlagbar ist. Der Blutglucosenachweis speziell mittels berührungsloser optischer Methoden ist im Stand der Technik an sich bekannt und wird daher hier nicht näher erläutert.

Der Stechantrieb 12 ermöglicht eine kontrollierte Vorwärts- und Rückziehbewegung des Stechelements 14 entlang einer Stechachse 22, wobei die Stechtiefe zweckmäßig im Bereich zwischen 1 und 2,5 mm zur Anpassung an verschiedene Hauttypen mittels einer Einstelleinheit 24 durch den Benutzer wählbar ist. Gegebenenfalls kann die Position der Hautoberfläche mittel einer Positioniereinheit 26 für das Körperteil 17 vorbestimmt werden.

Für eine mehrphasige Bewegungssteuerung umfasst der Stechantrieb 12 einen elektrisch arbeitenden Antriebsmotor 28 und einen damit vorgespannten, rein mechanisch arbeitenden Antriebsmechanismus 30. Der Antriebsmechanismus 30 steuert die schnelle Vorwärtsbewegung und eine erste schnelle Phase der Rückbewegung, während der Antriebsmotor 28 das Stechelement 14 über den Antriebsmechanismus 30 in einer zweiten Rückziehphase langsam aus der Haut zurückzieht. Auf diese Weise kann der Sammelvorgang optimiert und besonders benutzerfreundlich gestaltet werden.

Der mechanische Antriebsmechanismus 30 weist einen Spannrotor 32 und einen Antriebsrotor 34 auf, wobei die Rotoren über eine unter Vorspannung stehende Torsionsfeder 36 miteinander verbunden sind. Des Weiteren umfasst der Antriebsmechanismus 30 einen Kurven- bzw. Kulissentrieb 38, welcher die Rotationsbewegung des Antriebsrotors 34 über eine Steuerkurve 40 in eine Translations- bzw. Stechbewegung des angekoppelten Stechelements 14 umsetzt. Zu diesem Zweck greift ein an dem Antriebsrotor 34 abstehender Steuerarm 42 an seinem freien Ende über einen Kurvenreiter 44 in die umlaufende Steuerkurve 40 ein. Bei einer Drehung des Antriebsrotors 34 wird entsprechend der Kurvensteigung ein Hub erzeugt, wobei der Kurventrieb 38 über eine Linearführung 46 in dem Gerätegehäuse 48 geführt ist.

Die Relativdrehung der beiden Rotoren 32, 34 kann durch Anschlagelemente 50, 52 gegenseitig begrenzt werden, um die Vorspannung der Feder 36 aufzunehmen und den Antriebsrotor 34 in einer gewünschten Drehwinkelstellung zu stoppen. In einer vorbereitenden Spannphase wird der Antriebsrotor 34 drehfest gegenüber dem Gehäuse 48 arretiert, so dass die Feder 36 über den Spannrotor 32 durch Drehung des Motors 28 gespannt werden kann, bis die Ausgangsstellung der Anschlagelemente 50, 52 hergestellt ist. Die Arretierung des Antriebsrotors 34 wird in einer gegebenen Winkelstellung des Spannrotors 32 durch einen nicht gezeigten Auslöser aufgehoben, so dass der Antriebsrotor 34 augenblicklich federgetrieben dreht, bis das antriebsrotorseitige Anschlagstück 50 am anderen Ende der spannrotorseitigen Anschlagnut 52 anschlägt. Auf diese Weise kann ein Winkelbereich der Steuerkurve 40 abgefahren werden, um die Vorwärtsbewegung und die erste Rückziehphase der Rückziehbewegung sehr schnell auszuführen.

Weitere Einzelheiten eines geeigneten Antriebsmechanismus sind in der EP-A 1 669 028 beschrieben, die diesbezüglich durch Bezugnahme zum Gegenstand der vorliegenden Anmeldung gemacht wird.

Wie erwähnt, ist der Antriebsmotor 28 abtriebsseitig mit dem Spannrotor 32 gekoppelt, um in einer vorbereitenden Spannphase den Mechanismus 30 mit mechanischer Energie zu versorgen. Eine weitere wesentliche Funktion des Antriebsmotors 28 besteht in der kontrollierten langsamen Rückbewegung des Stechelements 14 während der zweiten Rückziehphase. Die Anschlagelemente 50, 52 werden dabei durch die verbleibende Federspannung in der oben beschriebenen Endstellung gehalten. Dadurch kann der Antriebsmechanismus 30 als Einheit weitergedreht werden, um den verbleibenden Abschnitt der Steuerkurve 40 abzufahren, wobei das Stechelement 14 mit definierter Rückziehgeschwindigkeit zurückgezogen wird. In dieser Phase kann das noch unter der Haut 16 befindliche Stechorgan 16 über die Sammelstruktur 18 aus der partiell freiwerdenden Stichwunde hinreichend Blut aufnehmen. Anschließend wird das aufgenommene Blut in einem Transferschritt durch eine geeignete Aktuation in bevorzugt weniger als 0,5 s auf das Testelement 20 übertragen. Zu diesem Zweck ist das Testelement 20 hinreichend nahe an der Aufnahmestruktur angeordnet, so dass der Flüssigkeitstransport unter Berücksichtigung der erreichbaren Transportgeschwindigkeit in der gegebenen Zeit erfolgt.

Für eine möglichst erfolgreiche und schmerzarme Blutaufnahme ist das in Fig. 2 gezeigte Stechprofil besonders vorteilhaft. Unter dem Begriff "Stechprofil" ist hierbei der Zeitverlauf der Stechbewegung zu verstehen, wie er als Funktion der Stechtiefe über der Zeit dargestellt ist.

In der Phase v der Vorwärtsbewegung trifft das Stechelement 114 mit seiner Spitze 16 bei t=0 mit einer hohen Geschwindigkeit auf der Haut auf und dringt in einem Zug bis zur gewünschten Einstichtiefe d vor. Diese Tiefe muss individuell optimiert werden, um durch die Epidermis hindurch die Blutkapillaren enthaltende Dermis zu erreichen. Die Dauer der Vorwärtsbewegung beträgt bevorzugt zwischen 0,3 bis 0,7 ms.

Sodann wird die Spitze 16 in der ersten Rückziehphase R1 um eine vorbestimmte Strecke Δd von ca. 0,5 mm auf eine unter der Hautoberfläche liegende Zwischenposition zurückgezogen. Bevorzugt liegt diese Rückziehposition im Stratum Corneum der Epidermis. Dieser erste Rückzug R1 sollte so rasch wie möglich erfolgen, weil das durch die scharfe Richtungsumkehr zu Schwingungen angeregte Stechelement 14 möglichst nicht zu viele Schwingungsperioden in der durchbluteten und innervierten Dermis ausführen sollte. Die kurz nach der Bewegungsumkehr erreichte maximale Rückziehgeschwindigkeit sollte daher mehr als 0,02 m/s betragen. Entsprechend wird die Dauer der ersten Rückziehphase im Bereich zwischen 0,3 und 1 ms begrenzt. Wie oben ausgeführt, wird ein einheitlicher harmonischer Bewegungsablauf in den Phasen v und R1 mittels des Antriebsmechanismus 30 erreicht.

Am Ende der Phase R1 wird der Rückzug des Stechelements 14 deutlich verlangsamt, so dass der Sammelvorgang während der anschließenden zweiten Rückziehphase R2 nicht kürzer als 0,4 s ist und vorzugsweise 0,5 bis 0,8 s in Anspruch nimmt. Der Verweildauer des Stechelements im eingestochenen Zustand in der Haut sollte jedenfalls höchstens ca. 1,5 s betragen, da alles darüber Hinausgehende vom Anwender als lästig empfunden wird. Um eine hinreichende Blutaufnahme in der Aufnahmestruktur 18 zu erreichen, sollte die Geschwindigkeit des Stechelements in der zweiten Rückziehphase im Wesentlichen konstant sein, wobei ein Wert zwischen 0,001 und 0,005 m/s zweckmäßig ist. Ein solcher langsamer Rückzug kann durch Beaufschlagung eines kompakten Antriebsmotors 28 mit einer mit einfachen Mitteln konstant gehaltenen Spannung energiesparend erreicht werden.

Das Stechprofil kann unabhängig von der Stechtiefe fest vorgegeben sein. Bei einem tieferen Einstich wird dann die in Fig. 2 gezeigte Kurve gleichsam unverändert nach oben verschoben. Alternativ kann es von Vorteil sein, den Geschwindigkeitsverlauf in Abhängigkeit einer wahlweise veränderten Stechtiefe so anzupassen, dass eine definierte Verweilzeit im eingestochenen Zustand erreicht wird.

## Patentansprüche

1. Vorrichtung zur Körperflüssigkeitsentnahme mit einem in die Haut eines Körperteils (17) einstechbaren Stechelement (14), das eine insbesondere als Kapillare wirksame Aufnahmestruktur (18) für beim Hauteinstich gewonnene Körperflüssigkeit aufweist, und einem Stechantrieb (12) für eine Vorwärts- und Rückziehbewegung des Stechelements (14), wobei die Dauer der Rückziehbewegung vorzugsweise um ein Vielfaches länger als die Dauer der Vorwärtsbewegung ist, **dadurch gekennzeichnet, dass** der Stechantrieb (12) dazu ausgebildet ist, das Stechelement (14) in einer ersten Rückziehphase (R1) der Rückziehbewegung mit einer maximalen Rückziehgeschwindigkeit von mehr als 0,02 m/s zu bewegen.

2. Vorrichtung nach Anspruch 1, **dadurch gekennzeichnet, dass** das Stechelement (14) während der ersten Rückziehphase (R1) um eine definierte erste Teilstrecke vorzugsweise von ca. 0,5 mm aus der tiefsten Einstechposition in eine unter der Hautoberfläche liegende Zwischenposition zurückgezogen wird.

3. Vorrichtung nach Anspruch 1 oder 2, **dadurch gekennzeichnet, dass** das Stechelement (14) während einer zweiten Rückziehphase (R2) um eine zweite Teilstrecke aus der Haut zurückgezogen wird, und dass die mittlere Geschwindigkeit des Stechelements in der zweiten Rückziehphase (R2) kleiner ist als in der ersten Rückziehphase (R1).

4. Vorrichtung nach einem der Ansprüche 1 bis 3, **dadurch gekennzeichnet, dass** die Dauer der Vorwärtsbewegung zwischen 0,3 bis 0,7 ms beträgt.

5. Vorrichtung nach einem der Ansprüche 1 bis 4, **dadurch gekennzeichnet, dass** die Dauer der ersten Rückziehphase (R1) zwischen 0,3 und 1 ms liegt.

6. Vorrichtung nach einem der Ansprüche 1 bis 5, **dadurch gekennzeichnet, dass** die Dauer der zweiten Rückziehphase (R2) nicht kürzer als 0,4 s ist und vorzugsweise 0,5 bis 0,8 s beträgt.

7. Vorrichtung nach einem der Ansprüche 1 bis 6, **dadurch gekennzeichnet, dass** der Bewegungsablauf des Stechelements (14) über den Stechantrieb (12) so gesteuert ist, dass die Verweildauer des Stechelements (14) im eingestochenen Zustand in der Haut im Bereich zwischen 0,5 bis 1,5 s liegt.

8. Vorrichtung zur Körperflüssigkeitsentnahme mit einem in die Haut eines Körperteils (16) einstechbaren Stechelement (14), das eine insbesondere als Kapillare wirksame Aufnahmestruktur (18) für beim Hauteinstich gewonnene Körperflüssigkeit aufweist, und einem Stechantrieb (12) für eine Vorwärts- und Rückziehbewegung des Stechelements (14), wobei die Dauer der Rückziehbewegung vorzugsweise um ein Vielfaches länger als die Dauer der Vorwärtsbewegung ist, **dadurch gekennzeichnet, dass** die Verweildauer des Stechelements (14) im eingestochenen Zustand in der Haut weniger als 1,5 s, vorzugsweise weniger als 1,2 s, und mehr als 0,5 s beträgt.

9. Vorrichtung nach einem der Ansprüche 1 bis 8, **dadurch gekennzeichnet, dass** die maximale Einstichtiefe (d) zwischen 1 und 2,5 mm einstellbar ist.

10. Vorrichtung nach einem der Ansprüche 1 bis 9, **dadurch gekennzeichnet, dass** der Geschwindigkeitsverlauf bei der Rückbewegung des Stechelements (14) nach Maßgabe einer veränderlichen Stechtiefe so angepasst ist, dass das Stechelement (14) für eine vorbestimmte Verweilzeit vorzugsweise zwischen 0,5 und 1,5 s in der Haut eingestochen bleibt.

11. Vorrichtung nach einem der Ansprüche 1 bis 10, **dadurch gekennzeichnet, dass** der Geschwindigkeitsverlauf bei der Rückbewegung des Stechelements (14) unabhängig von der Stechtiefe vorgegeben ist.

12. Vorrichtung nach einem der Ansprüche 1 bis 11, **dadurch gekennzeichnet, dass** die Geschwindigkeit des Stechelements (14) in einer an die erste Rückziehphase anschließenden zweiten Rückziehphase (R2) im Wesentlichen konstant ist.

13. Vorrichtung nach einem der Ansprüche 1 bis 12, **dadurch gekennzeichnet, dass** die maximale Geschwindigkeit des Stechelements (14) in der zweiten Rückziehphase (R2) zwischen 0,001 und 0,005 m/s beträgt.

14. Vorrichtung nach einem der Ansprüche 1 bis 13, **gekennzeichnet durch** ein mit Körperflüssigkeit aus der Aufnahmestruktur (18) beaufschlagbares Testelement (20) zum Nachweis eines Analyten in der Körperflüssigkeit.

15. Vorrichtung nach Anspruch 14, **dadurch gekennzeichnet, dass** die Gesamtdauer vom Beginn der Rückziehbewegung des Stechelements (14) bis zur Beaufschlagung des Testelements (20) mit der Körperflüssigkeit weniger als 5 s, vorzugsweise weniger als 1 bis 2 s beträgt.

16. Vorrichtung zur Körperflüssigkeitsentnahme mit einem in die Haut eines Körperteils (17) einstechbaren Stechelement (14), das eine insbesondere als Kapillare wirksame Aufnahmestruktur (18) für beim Hauteinstich gewonnene Körperflüssigkeit aufweist, und einem Stechantrieb (12) für eine Vorwärts- und Rückziehbewegung des Stechelements (14), **dadurch gekennzeichnet, dass** ein für den Nachweis eines Analyten in der Körperflüssigkeit ausgebildetes Testelement (20) so angeordnet ist, dass die Transferzeit zur Übertragung der Körperflüssigkeit aus der Aufnahmestruktur (18) auf das Testelement (20) weniger als 1,5s, bevorzugt weniger als 0,5s beträgt.

17. Vorrichtung nach einem der Ansprüche 1 bis 16, **dadurch gekennzeichnet, dass** das Stechelement (14) einen zumindest an einem distalen Endabschnitt beim Hauteinstich in Kontakt mit der Körperflüssigkeit bringbaren, insbesondere rinnen- oder schlitzförmigen Kanal als Aufnahmestruktur (18) aufweist.

18. Vorrichtung zur Körperflüssigkeitsentnahme insbesondere nach einem der vorhergehenden Ansprüche umfassend ein in die Haut eines Körperteils (17) einstechbares Stechelement (14), das eine insbesondere als Kapillare wirksame Aufnahmestruktur (18) für beim Hauteinstich gewonnene Körperflüssigkeit aufweist, und einen Stechantrieb (12) für eine Vorwärts- und Rückziehbewegung des Stechelements (14), wobei der Stechantrieb (12) einen Antriebsmotor (28) und einen mechanisch arbeitenden Antriebsmechanismus (30) aufweist, **dadurch gekennzeichnet, dass** der Antriebsmechanismus (30) die Vorwärtsbewegung und Rückziehbewegung während einer ersten Rückziehphase (R1) des Stechelements (14) steuert, und dass der Antriebsmotor (28) die Rückziehbewegung des Stechelements (14) in einer zweiten Rückziehphase (R2) bewirkt.

19. Vorrichtung nach einem der Ansprüche 1 bis 18, **dadurch gekennzeichnet, dass** der Antriebsmotor (28) den Antriebsmechanismus (30) zur selbsttätigen Bewegungssteuerung mit mechanischer Energie versorgt.

20. Vorrichtung nach einem der Ansprüche 1 bis 19, **dadurch gekennzeichnet, dass** der Antriebsmotor (28) das Stechelement (14) über den Antriebsmechanismus (30) in der zweiten Rückziehphase (R2) zurückzieht.

21. Vorrichtung nach einem der Ansprüche 1 bis 20, **dadurch gekennzeichnet, dass** der Antriebsmechanismus (30) eine mittels einer Feder (36) angetriebene Kurvensteuerung (40) aufweist.

22. Verfahren zur Körperflüssigkeitsentnahme bei welchem ein Stechelement (14) in die Haut eines Körperteils (17) eingestochen wird und die beim Hauteinstich gewonnene Körperflüssigkeit in einer insbesondere als Kapillare wirksamen Aufnahmestruktur (18) des Stechelements (14) aufgenommen wird, wobei durch einen Stechantrieb (12) eine Vorwärts- und Rückziehbewegung des Stechelements (14) so gesteuert wird, dass die Dauer der Rückziehbewegung vorzugsweise um ein Vielfaches länger als die Dauer der Vorwärtsbewegung ist, **dadurch gekennzeichnet, dass** das Stechelement (14) in einer ersten Rückziehphase (R1) der Rückziehbewegung mit einer maximalen Rückziehgeschwindigkeit von mehr als 0,02 m/s zurück bewegt wird.
